(19) Europäisches Patentamt
European Patent Office
Office européen des brevets

(11) Publication number: **0 385 686 A2**

# (12) EUROPEAN PATENT APPLICATION

(21) Application number: 90302001.4

(22) Date of filing: 26.02.90

(51) Int. Cl.[5] C08F 8/32, C08F 8/44, A61K 31/74

(30) Priority: 28.02.89 GB 8904489

(43) Date of publication of application: 05.09.90 Bulletin 90/36

(84) Designated Contracting States: CH DE FR GB IT LI NL

(71) Applicant: SMITH KLINE & FRENCH LABORATORIES LIMITED
Mundells
Welwyn Garden City Hertfordshire, AL7 1EY(GB)

(72) Inventor: Hickey, Deirdre Mary Bernadette
Smith Kline & French Research Ltd., The Frythe
Welwyn, Hertfordshire AL6 9AR(GB)
Inventor: Whittaker, Caroline Marie
Smith Kline & French Research Ltd., The Frythe
Welwyn, Hertfordshire AL6 9AR(GB)
Inventor: Jaxa-Chamiec, Albert Andrzej
22 Beacon Way, Rickmansworth,
Hertfordshire WD3 2PE(GB)

(74) Representative: Giddings, Peter John, Dr. et al
SmithKline Beecham, Corporate Patents, Mundells
Welwyn Garden City, Hertfordshire AL7 1EY(GB)

## (54) Compounds.

(57) Polystyrene polymers of structure :

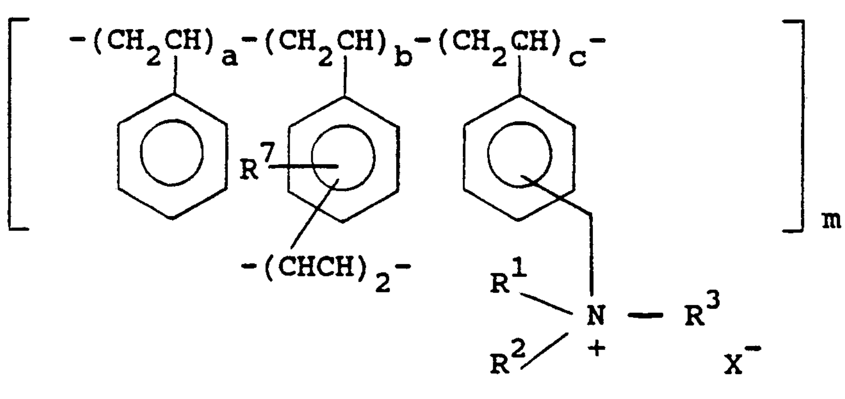

(I)

in which, $R^1$ and $R^2$ are the same or different and are each $C_{1-4}$ alkyl; $R^3$ is $(CH_2)_nR^4$ in which $R^4$ is $C_{3-6}$ cycloalkyl or a bicyclic carbocyclic ring of up to 10 carbon atoms and n is 1 to 12, or $R^3$ is $(CH_2)_nCH = R^5R^6$ in which n is 1 to 12 and $R^5$ and $R^6$ together form a $C_{3-6}$ cycloalkyl ring or a bicyclic carbocyclic ring of up to 10 carbon atoms; $R^7$ is hydrogen or a group $CH_2\overset{+}{N}R^1R^2R^3X^-$; $X^-$ is a counter ion; a, b and c are numbers which indicate the relative molar percentages of the units present in said polymer, (b) being from about 1 to about 10 molar percent, and (c) being from about 30 to about 98 molar percent; m is a number indicating the degree of polymerisation of said polymer, processes for their preparation, compositions containing them and their use in therapy as bile acid sequestering agents.

EP 0 385 686 A2

# COMPOUNDS

The present invention relates to novel polystyrene anion exchange resins, processes for their preparation, pharmaceutical compositions containing them and their use in the lowering of plasma cholesterol levels in humans.

Coronary Heart Disease (CHD) is one of the most serious health problems of contemporary society. Worldwide epidemiological studies have shown that the incidence of CHD is related to a number of independent risk factors, in particular, for example, high concentrations of serum cholesterol (hypercholesterolaemia). Such adverse factors lead to atherosclerosis, and ultimately, in severe cases, intermittent claudication, cerebrovascular insufficiency, thrombosis and cardiac arrest.

It is known that ion exchange resins, in particular polystyrene resins can be used as sequestering agents to bind non-absorbed bile acids and salts in the intestinal tract, forming complexes which are then excreted in the faeces. This sequestering leads to a decrease in the amount of bile acids returning to the liver via enterohepatic circulation. The synthesis of replacement bile acids from hepatic cholesterol depletes hepatic cholesterol, regulates hepatic LDL receptors and consequently reduces plasma cholesterol levels. Such sequestering resins have been recognised as useful for the treatment of hypercholesterolaemia. In addition, it is now proven that reducing serum cholesterol with bile acid sequestrants has a beneficial effect on protecting against the occurrence of coronary heart disease.

One particular agent which is currently used to lower serum cholesterol levels in humans by binding bile acids in the intestinal tract is cholestyramine. Cholestyramine is a cross-linked anion exchange polystyrene resin bearing an ionisable trimethylammonium group bound to the polymer backbone. However, the use of this agent is associated with a number of undesirable side-effects, for example, it is unpalatable and must be taken in high doses and causes, in some cases, bloating, constipation and other gut side-effects. In addition, its ability to bind bile acids is inefficient with respect to the amounts of resin which it is necessary to use.

The present invention provides compounds which overcome the disadvantages of this known sequestering agent and provides improved bile acid sequestering agents which are useful for lowering serum cholesterol levels in humans.

The present invention therefore provides in a first aspect, polystyrene polymers of structure (I) :

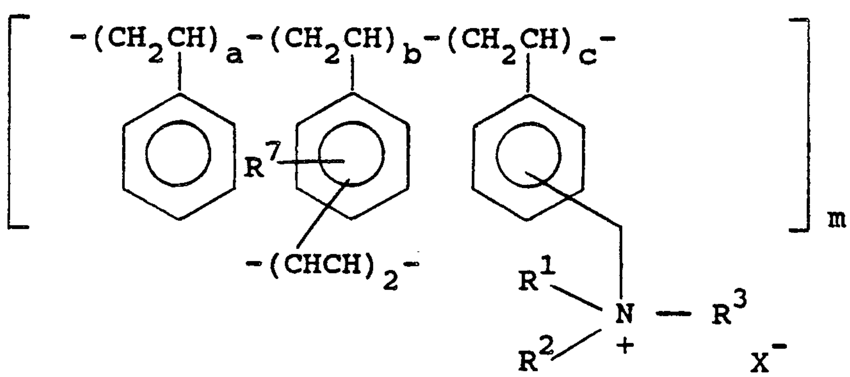

(I)

in which,

$R^1$ and $R^2$ are the same or different and are each $C_{1-4}$ alkyl;

$R^3$ is $(CH_2)_nR^4$ in which $R^4$ is $C_{3-6}$ cycloalkyl or a bicyclic carbocyclic ring of up to 10 carbon atoms and n is 1 to 12, or $R^3$ is $(CH_2)_nCH = R^5R^6$ in which n is 1 to 12 and $R^5$ and $R^6$ together form a $C_{3-6}$ cycloalkyl ring or a bicyclic carbocyclic ring of up to 10 carbon atoms;

$R^7$ is hydrogen or a group $CH_2\overset{+}{N}R^1R^2R^3X^-$;

$X^-$ is a counter ion;

a, b and c are numbers which indicate the relative molar percentages of the units present in said polymer, (b) being from about 1 to about 10 molar percent, and (c) being from about 30 to about 98 molar percent;

m is a number indicating the degree of polymerisation of said polymer.

Suitably the groups $R^1$ and $R^2$ are the same or different; preferably they are the same; most preferably $R^1$ and $R^2$ are both methyl.

Suitably $R^3$ is a group $(CH_2)_nCH = R^5R^6$ in which n, $R^5$ and $R^6$ are as hereinbefore described. More suitably $R^3$ is a group $(CH_2)_nCH = R^5R^6$ in which $R^5$ and $R^6$ together form a cyclohexyl ring. Preferably $R^3$ is a group $(CH_2)_nR^4$ in which $R^4$ is $C_{3-6}$cycloalkyl or a bicyclic carbocylic ring of up to 10 carbon atoms, and n is 1 to 12. More preferably $R^3$ is a group $(CH_2)_nR^4$ in which $R^4$ is a $C_{3-6}$cycloalkyl group, in particular a cyclohexyl group.

Suitably (b) is from about 1 to about 10 molar percent of said polymer, preferably (b) is from about 1 to about 8 molar percent, most preferably from about 1 to about 4 molar percent of said polymer.

m is a number indicating the degree of polymerisation of the polymer. Owing to the three dimensional cross-linkage, precise figures cannot be given for m, but in any case will be, in general, greater than 1,000.

Suitably $X^-$ is a physiologically acceptable counter ion such as a halide, in particular chloride, sulphate, phosphate, bicarbonate, carbonate, formate, acetate, sulphonate, propionate, malonate, succinate, malate, tartrate, citrate, maleate, fumarate, ascorbate, glucuronate or the anion of an amino acid such as aspartic or glutamic acid. More suitably $X^-$ is a chloride, sulphate or phosphate ion; preferably $X^-$ is a chloride ion.

The polystyrene polymers of the present invention are also characterised by their total exchange capacity i.e. the theoretical maximum capacity of the resin if each counter ion were to be exchanged with bile acid. In this specification the total exchange capacity is defined in terms of the number of milliequivalents of counter ion per gram of dry weight of polymer.

Suitable total exchange capacities are in the range of, for example where the counter ion $X^-$ is chloride, from 1.5 to 3.5 meq $Cl^-$ per gram of resin. Preferred within this range are polymers having a total exchange capacity of between 2 and 3 meq $Cl^-$/gram of resin.

The polystyrene polymers of the present invention can be prepared by processes analogous to those known in the art. The present invention therefore provides, in a further aspect, a process for preparing the polystyrene polymers of structure (I) which comprises :

(a) reaction of a polymer of structure (II)

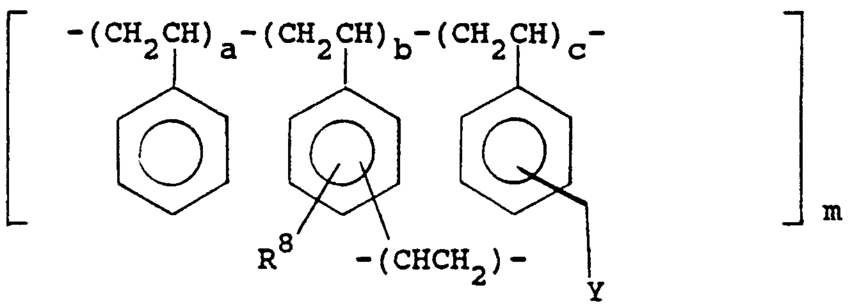

(II)

in which a, b, c and m are as described for structure (I) $R^8$ is $CH_2Y$ and Y is a group displaceable by an amine, with an amine of structure $R^1R^2NR^3$ (III) in which $R^1$ to $R^3$ are as described for structure (I); or

(b) reaction of a polymer of structure (IV)

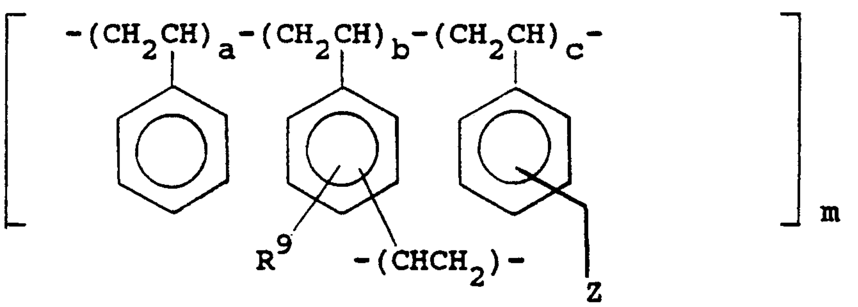

(IV)

in which a, b, c and m are as described for structure (I) $R^9$ is $CH_2Z$ and Z is $NR^1R^2$ or $NR^1R^3$ in which $R^1$ to $R^3$ are as described for structure (I) with a compound of structure $R^4Y$ (V) in which $R^4$ is $R^3$ when Z is $NR^1R^2$ or a group $R^2$ when Z is $NR^1R^3$, and Y is a group displaceable by an amine.

The reaction between a polymer of structure (II) and a compound of structure (III) can be carried out in a suitable solvent at elevated temperature. Suitable solvents included for example, a $C_{1-4}$alkanol, dimethylformamide, N-methylpyrrolidone or tetrahydrofuran. Preferably the reaction is carried out in dimethylformamide at a temperature of between about 60° and 80° for a period of up to 24 hours or until the reaction is complete.

The reaction between a polymer of structure (IV) and a compound of structure (V) can be carried out in a suitable inert solvent such as a $C_{1-4}$alkanol, dimethylformamide, tetrahydrofuran or N-methylpyrrolidone at elevated temperature.

The intermediate polymers of structure (II) are available commercially or can be prepared from readily available materials by methods known to those skilled in the art. For example polymers of structure (II) in which Y is chlorine can be prepared by reaction of chloromethylstyrene, styrene and divinyl benzene in an aqueous suspension comprising polyvinyl alcohol in the presence of an initiator at elevated temperature. Suitable initiators will be apparent to those skilled in the art and include, in particular azobisisobutyronitrile.

The intermediate polymers of structure (IV) can be prepared from the polymers of structure (II) by reaction with an amine of structure $HNR^1R^2$ or $HNR^1R^3$ in which $R^1$ to $R^3$ are as described for structure (I) under the same or similar conditions as indicated for the reaction of a compound of structure (II) and a compound of structure (III).

Alternatively, the intermediate polymers of structure (IV) can be prepared directly from polystyrene by methods analogous to those known in the art, for example where $Z^{\ominus}$ is chlorine by chloromethylation of polystyrene.

The polystyrene polymers of structure (I) have been found to bind bile acids both in in vitro and in in vivo models in that they increase the amount of bile acids in the faeces. As indicated earlier it is recognised that removal of bile acids from the intestinal tract in this way lowers serum cholesterol levels and also has a beneficial effect on protecting against atherosclerosis and its dependent clinical conditions. The present invention therefore provides in a further aspect, the use of polystyrene polymers of structure (I) in therapy, in particular for the lowering of serum cholesterol levels in mammals, including humans. In addition the polymers of structure (I) are expected to be of use in protecting against atherosclerosis and its sequelae.

When used in therapy the polystyrene polymers of structure (I) are in general administered in a pharmaceutical composition.

In a still further aspect of the present invention there is therefore provided a pharmaceutical composition comprising a polystyrene polymer of structure (I) in association with a pharmaceutically acceptable carrier.

The compositions of the present invention can be prepared by techniques well known to those skilled in the art of pharmacy and include all those known for the formulation of polystyrene polymers for human use.

The polymers are preferably administered as formulations in admixture with one or more conventional pharmaceutical excipients which are physically and chemically compatible with the polymer, which are nontoxic, are without deleterious side-effects but which confer appropriate properties on the dosage form.

In general, for liquid formulations aqueous pharmaceutically acceptable carriers such as water or aqueous dilute ethanol, propylene glycol, polyethylene glycol or glycerol or sorbitol solutions are preferred. Such formulations can also include flavouring and sweetening agents such as sucrose, fructose, invert sugar, cocoa, citric acid, ascorbic acid, fruit juices etc. In general, digestible oil or fat based carriers should be avoided or minimised as they contribute to the condition sought to be alleviated by use of the polymers. They are also subject to absorption by the polymers during prolonged contact, thus reducing the capacity of the polymer to absorb bile acids after administration.

The polymers can also be prepared as concentrates, for dilution prior to administration, and as formulations suitable for direct oral administration. They can be administered orally ad libitum, on a relatively continuous basis for example by dispersing the polymer in drinks or food.

Preferably, the polymers are administered in tablet form or in gelatin capsules containing solid particulate polymer or an aqueous or semi-aqueous suspension of solid polymer containing a suitable suspending agent.

Preferably the polymer is administered in unit dosage form, each dosage unit containing preferably from 0.5 g to 1 g of polymer.

The daily dosage regimen for an adult patient may be, for example, an oral dose of between 1 and 10 g, preferably 1-5 g the compound being administered 1 to 4 times a day. Suitably the compound is administered for a period of continuous therapy of one month or more sufficient to achieve the required reduction in serum cholesterol levels.

In addition the polymers of the present invention can be co-administered (together or sequentially) with further active ingredients such as HMGCoA reductase inhibitors and other hypocholesterolaemic agents, and other drugs for the treatment of cardiovascular diseases.

The following biological data and examples indicate the properties and preparation of the polymers of the present invention. Temperatures are recorded in degrees centigrade. The exchange capacity of the ammonium substituted resins was determined by elemental analysis and/or potentiometric titration of

chloride ion, and are expressed as milli equivalents of exchangeable chloride ion per gram of dry resin weight.

## Example 1

To a suspension of chloromethylstyrene-styrenedivinylbenzene copolymer (1% w/w divinylbenzene, 4.09 meq Cl/g) (2 g) in dimethylformamide (DMF) (30 ml), was added N,N-dimethylcyclohexylamine (2.0 g) and the mixture stirred at 70° for 7 hours. The polymer was filtered off and washed with DMF, 50:50 DMF:methanol, methanol, and ether giving N,N-dimethyl-N-cyclohexylaminomethylsubstituted polystyrene chloride salt, as off-white resin beads (2.88 g, 95%) (exchange capacity = 2.60 meq $Cl^-$/g).

## Example 2

(a) To a solution of cyclohexanecarboxylic acid (30 g) in dry tetrahydrofuran (300 ml) triethylamine (40.7 ml) was added and the solution was cooled to 5-10°. Ethyl chloroformate (27.9 ml) was added over 40 minutes keeping the temperature at 10°. The mixture was stirred at 10° for 40 minutes. 33% Dimethylamine in ethanol (83 ml) was added over 40 minutes keeping the temperature at 10°. The mixture was then stirred at room temperature for 19 hours, filtered to remove inorganics, and the filtrate evaporated to dryness. The resulting oil was dissolved in chloroform (300 ml) and washed with 10% aqueous sodium carbonate solution, water, then dried with anhydrous magnesium sulphate and evaporated to dryness. The resulting oil was distilled at 0.1 Torr to give N,N-dimethylcyclohexanecarboxamide as a colourless oil (22.43 g, 61.7%).

(b) To a suspension of $LiA1H_4$ (8.0 g) in dry tetrahydrofuran (250 ml) under nitrogen, 22 g of the above compound in dry tetrahydrofuran (50 ml) was added over 0.5 minutes. The reaction mixture was stirred at room temperature for 4 hours, then cooled and water (8 ml), 15% sodium hydroxide solution (8 ml), and water (24 ml) were sequentially added. After the mixture was stirred at room temperature for 1 hour, the white precipitate was filtered off and the filtrate evaporated to dryness. The residue was dissolved in chloroform (250 ml) and washed with 2N sodium hydroxide, water, then dried with anhydrous magnesium sulphate and evaporated to dryness. The resulting oil was distilled to give N,N-dimethyl-N-cyclohexylmethylamine as a colourless oil (b.p. 89-94°, 0.1 Torr) (14.1 g, 70.5%).

(c) The above amine (6.6g) was reacted with 1% cross-linked chloromethylated polystyrene (5 g, 18.6 mmoles) in DMF (75 ml) at 70° for 24 hours, to give after washing as in Example 1, N,N-dimethyl-N-cyclohexylmethylammonium-substituted polystyrene, chloride salt, as off-white resin beads (7.4 g, 97%) (2.56 meq $Cl^-$/g).

## Example 3

(a) A solution of cyclohexylacetic acid (18.8 g), triethylamine (23 ml) in dry tetrahydrofuran (200 ml) was reacted with ethyl chloroformate (15.8 ml) and then 33% dimethylamine in ethanol (47 ml) by a method analogous to Example 2a. The reaction mixture was stirred at room temperature overnight before it was filtered. The filtrate was evaporated to dryness and the resulting oil was dissolved in ethyl acetate (100 ml) and washed with 10% aqueous sodium carbonate, water, then dried with anhydrous magnesium sulphate and evaporated to dryness to give N,N-dimethylcyclohexylacetamide as an oil (17.91 g, 80%).

(b) The above compound (17.2 g) was reduced with lithium aluminium hydride (5.8 g) in dry tetrahydrofuran (200 ml) by a method analogous to Example 2b. The reaction mixture was cooled and triethanolamine (23 g) was added over 1 hour. Water (2.75 g) was added giving a white precipitate which was filtered off after 1 hour. The filtrate was evaporated to dryness and purified by silica gel chromatography, eluting with chloroform: methanol to give 2-cyclohexyl-N,N-dimethylethylamine as a colourless oil (9.01 g, 57%).

(c) The above amine (5.2 g) was reacted with 1% cross-linked chloromethylated polystyrene (3 g, 11.16 mmoles) in DMF (75 ml) at 70° for 24 hours, to give after washing as in Example 1 N,N-dimethyl-N-(2-cyclohexylethyl)ammoniomethyl-substituted polystyrene, chloride salt, as off-white resin beads (4.53, 96%) (exchange capacity 2.49 meq $Cl^-$/g).

Example 4

(a) To a solution of cyclohexanepropionic acid (70 g) and triethylamine (78 ml) in dry tetrahydrofuran (700 ml), ethylchloroformate (53.3 ml) and then 33% dimethylamine in ethanol (162 ml) were added in a method analogous to Example 2a. The reaction mixture was stirred at room temperature for 3 hours and was then filtered and the filtrate evaporated to dryness. The residue was dissolved in chloroform and washed with 2N aqueous sodium hydroxide solution, water, then dried with anhydrous magnesium sulphate and evaporated to dryness. The resulting oil was purified by distillation to give 3-cyclohexyl-N,N-dimethyl propionamide as a colourless oil, (63 g, 77%).

(b) The above compound (62.5 g) was reduced with lithium aluminium hydride (19.41 g) in dry tetrahydrofuran (250 ml) by a method analogous to Example 2b. The work up was comparable to that used in Example 2b using water (19.4 ml), 15% sodium hydroxide (19.4 ml), water (58.2 ml). After the mixture was stirred at room temperature for 1 hour it was filtered and the filtrate was evaporated to dryness. The resulting oil was distilled to give 3-cyclohexyl-N,N-dimethylpropylamine as a colourless oil (bp 69-79°, 0.3 Torr), (47.34 g, 82%).

(c) The above amine (47 g) was reacted with 1% cross-linked chloromethylated polystyrene (30 g, 124.5 mmoles) in dimethylformamide (600 ml) at 50° for 66 hours, to give after washing as in Example 1, N,N-dimethyl-N-(3-cyclohexylpropyl)ammoniomethyl-substituted polystyrene, chloride salt, as off-white resin beads, (50.7 g, 99%) (exchange capacity 2.34 meq $Cl^-$/g).

Example 5

(a) To a solution of cyclohexanebutyric acid (15.0 g) and triethylamine (14.7 ml) in dry tetrahydrofuran (150 ml), ethyl chloroformate (10.1 ml) and then 33% dimethylamine in ethanol (32 ml) were added in a method analogous to Example 2a. The reaction was stirred at room temperature for 3 hours before it was filtered. The filtrate was evaporated to dryness and the residue was dissolved in chloroform (200 ml), washed with 10% aqueous sodium carbonate solution, water, then dried with anhydrous magnesium sulphate and evaporated to dryness. The resulting oil was purified by silica gel chromatography eluting with 10:1 $CHCl_3$:MeOH to give 4-cyclohexyl-N,N-dimethylbutyramide as an oil (14.82 g, 85%).

(b) The above amide (14.7 g) was reduced with lithium aluminium hydride (4.24 g) in dry tetrahydrofuran (200 ml) in a method analogous to Example 2b. The work up was comparable to that used in Example 3b using triethanolamine (16.7 g) and water (2.0 g). After stirring the white solid was removed by filtration and the filtrate was evaporated to dryness. The resulting oil was purified by silica gel chromatography eluted with 10:1 $CHCl_3$:MeOH to give 4-cyclohexyl-N,N-dimethylbutylamine as an oil (4.5 g, 32%).

(c) The above amine (2 g) was reacted with 1% cross-linked chloromethylated polystyrene (2 g, 7.44 mmoles) in dimethylformamide (75 ml) at 70° for 20 hours to give, after washing as in Example 1, N,N-dimethyl-N-(4-cyclohexylbutyl)ammoniomethyl-substituted polystyrene, chloride salt, as off-white resin beads (2.92 g, 87%) (exchange capacity 2.34 meq $Cl^-$/g).

Example 6

(a) 8-Bromooctanoic acid (90 g) and triphenylphosphine (105.8 g) were mixed in dry acetonitrile (700 ml) and refluxed for 24 hours. The reaction mixture was evaporated to dryness and the oil was stirred under anhydrous diethyl ether (200 ml). The ether was decanted off and more diethyl ether added, giving a grey solid. This was collected by filtration and washed with diethyl ether to give 7-carboxyheptyltriphenyl-phosphonium bromide as a grey solid, m.p. 120-122°, (172 g, 88%).

(b) To a mixture of potassium t-butoxide (38.1 g) in anhydrous diethyl ether (750 ml) under $N_2$ 7-carboxyheptyltriphenyl phosphonium bromide (75 g) was added. The mixture was heated at reflux temperature for 2 hours and the ether was then removed by distillation. Cyclohexanone (30 g) was added dropwise, maintaining reflux, to the slurry and after 0.5 hour anhydrous diethyl ether (250 ml) was added to aid stirring. The reaction mixture was refluxed for 3 hours and then the ether was removed by distillation. The mixture was cooled, 2N hydrochloric acid was added, and the aqueous solution extracted with chloroform. The chloroform solution was extracted with 1N sodium hydroxide. The basic extracts were acidified with 2N hydrochloric acid and extracted with chloroform and the organic extracts dried with

anhydrous magnesium sulphate and evaporated to dryness. The resulting oil was purified by silica gel chromatography ($CHCl_3$:MeOH gradient as eluent) to give 8-cyclohexylidene-1-octanoic acid, as an orange oil (23.1 g, 67%).

(c) To a mixture of the above carboxylic acid (33 g) in dry tetrahydrofuran (500 ml), triethylamine (24.6 ml) was added and the mixture was cooled to 5° in an ice bath. Ethyl chloroformate (16.9 ml) was added over 10 minutes keeping the temperature at about 10°. The reaction was stirred at 10° for 1 hour and then 33% dimethylamine in ethanol (200 ml) was added over 15 minutes keeping the temperature at about 15°. The reaction was stirred at room temperature for 3 hours and evaporated to dryness. The resulting oil was dissolved in dichloromethane (250 ml) and washed with 1N sodium hydroxide, water, then dried with anhydrous magnesium sulphate, and evaporated to dryness. The resulting oil was purified by silica gel chromatography ($CH_2Cl_2$:MeOH gradient as eluent) to give 8-cyclohexylidene-I-N,N-dimethyloctanamide as oil (16.50 g, 64%).

(d) To a suspension of lithium aluminium hydride (3.7 g) in dry tetrahydrofuran (200 ml) under nitrogen the above amide (16.4 g) in dry tetrahydrofuran (20 ml) was added over 15 minutes. The reaction was cooled as the temperature rose to 40°. The reaction was stirred at room temperature for 1.5 hours and then refluxed for 1 hour. The reaction was cooled to 0° and water (3.7 ml), 15% sodium hydroxide (3.7 ml), and water (11.1 ml) were sequentially added. When a thick white precipitate had formed the reaction was filtered and evaporated to dryness. (This was combined with the product from a reduction of the same amide (10.2 g) with lithium aluminium hydride (2.3 g) by a similar method). The oil was dissolved in diethyl ether (250 ml) and extracted with 2N HCl. The aqueous layer was basified with 2N NaOH and extracted with dichloromethane. The organic layer was washed with water, dried with anhydrous magnesium sulphate and evaporated to dryness to give an oil which was purified by distillation to give 8-cyclohexylidene-N,N-dimethyloctylamine as colourless oil, bp 110-114°, 0.05 Torr, (19.2 g, 76.5%).

(e) The above amine (5.6 g) was reacted with 1% cross-linked chloromethylated polystyrene (3 g, 12.45 mmoles) in dimethylformamide (75 ml) at 70° for 20 hours, to give, after washing as in Example 1, N,N-dimethyl-N-(8-cyclohexylideneoctyl)ammoniomethyl-substituted polystyrene, chloride salt, off-white resin beads (5.56 g, 94%) (exchange capacity = 2.10 meq $Cl^-$/g).

## Example 7

(a) 8-Cyclohexylidine-N,N-dimethyloctylamine (Example 6d) (13.5 g) was hydrogenated in ethanol (100 ml) at 40 p.s.i. in the presence of 10% Pd on C (1.0 g) at room temperature for 4 hours. The reaction mixture was filtered and evaporated to dryness. The resulting oil was purified by distillation to give 8-cyclohexyl-N,N-dimethyloctylamine as a colourless oil, bp 138-141°, 0.1 Torr, (12.9 g, 96%).

(b) The above amine (6 g) was reacted with 1% cross-linked chloromethylated polystyrene (3 g, 12.45 mmoles) in dimethylformamide (75 ml) at 70° for 20 hours to give, after washing as in Example 1, N,N-dimethyl-N-(8-cyclohexyloctyl)ammoniomethyl-substituted polystyrene, chloride salt, as off-white resin beads. (5.68 g, 95%) (exchange capacity 2.12 meq $Cl^-$/g).

## Example 8

(a) To a washed suspension of sodium hydride (12.6 g) in dry dimethylformamide (200 ml) triethylphosphonoacetate (52 ml) was added under nitrogen over one hour. The mixture was stirred at room temperature for 90 minutes and then 2-decalone (20 g) in dry dimethylformamide (20 ml) was added over 5 minutes. The reaction was stirred at room temperature for 2 hours and at 60° for 2 hours. The reaction was cooled and then poured onto ice water and evaporated to dryness. The residue was dissolved in ethyl acetate, washed with water, dried with anhydrous magnesium sulphate and evaporated to dryness. The resulting oil was purified by chromatography on silica gel to give ethyl dehydrodecalin-2-acetate as an oil (mixture of isomers) (16.1 g, 55%).

(b) A solution of the above mixture of esters (25 g) in ethanol (100 ml) and 2N sodium hydroxide (100 ml) was refluxed for 0.5 hour, cooled and then evaporated to remove ethanol. The aqueous solution was then acidified with 2N hydrochloric acid to pH 5 and extracted with chloroform, washed with water, dried over anhydrous magnesium sulphate and evaporated to dryness to give the corresponding mixture of acids [19.6 g, 90%].

(c) This mixture of acids (19 g) was treated with triethylamine, ethyl chloroformate and dimethylamine in dry tetrahydrofuran in the same manner as Example 2a to give the corresponding N,N-dimethyl amide(s)

as an oil (15.95, 74%).

(d) Reduction of this mixture of amides (14.75 g) with lithium aluminium hydride in tetrahydrofuran gave the corresponding N-N-dimethylamine(s) as a yellow oil (13.71 g, 99%).

(e) The above dehydro 2-(2-dimethylaminoethyl)decalin (13.6 g) was hydrogenated in ethanol (100 ml) at 45 p.s.i. at 35° in the presence of 10% palladium on charcoal catalyst (1.36 g) for 16 hours. The reaction mixture was filtered and evaporated to dryness. The resulting oil was purified by distillation in a Kugel Rohr at 250°/0.05 Torr to give N,N-dimethyl-2-(2-perhydronaphthyl)ethylamine a colourless oil (7.46 g, 55%).

(f) The above amine (5.8 g) was reacted with 1% cross-linked chloromethylated polystyrene (3.0 g, 11.6 mmoles) in dimethylformamide (75 ml) at 70° for 20 hours, to give after washing as in Example 1, N,N-dimethyl-N-[2-(2-perhyrdonaphthyl)ethyl]ammoniomethyl-substituted polystyrene, chloride salt, as off-white resin beads (5.11 g, 96.4%) (exchange capacity 2.14 meq $Cl^-$/g).

## Example A

A chewable tablet composition can be prepared from the following :

| | mg/tablet |
|---|---|
| Compound of Example 6: | 1250 |
| Silicon dioxide | 15 |
| Microcrystalline cellulose | 280 |
| Sorbitol | 445 |
| Lactose | 450 |
| Sweetener | 5 |
| Peppermint | 30 |
| Magnesium Stearate | 25 |
| | 2500 mg |

## Example B

A food additive composition, for example, a sachet for reconstitution or mixing with food, is prepared by incorporating into a powder formulation compound of Example 6 (250 mg), sodium carboxymethylcellulose (50 mg), sucrose (2400 mg) and flavours (50 mg).

## Binding Data

In the binding assays the resins used contained 5-15% $H_2O$ w/w and all weights used in the assays refer to these undried materials.

### 1. Equilibrium binding of Bile Acids (as sodium salts) to resins

The compounds of the examples (10 mg/tube) were incubated with a range of $^{14}C$ radiolabelled sodium taurochenodeoxycholate (TCDC) and sodium glycocholate (GC) solutions (0.05 mM-24 mM) of known specific activity, in Krebs Henseleit buffer (2.5 ml final volume). The resin was allowed to equilibrate with the bile salt solution for a period of three hours at 37°C in a shaking water bath. The resin was removed from the incubation medium by centrifugation (15 minutes at 3,000 g). The radioactivity of the supernatant was determined, and from this the free and bound quantities of bile salt were calculated. These were expressed as moles of bile salt bound per gram of resin.

Results

The compounds of examples at a concentration of 5.7 mM GC were found to bind in the range of from 0.62 to 0.95 mmoles GC per gram weight of resin; and at a concentration of 5.7 mM TCDC were found to bind in the range of from 0.24 to 1.37 mmoles TCDC per gram weight of resin.

DATA

In vitro Dissociation assay

The following assay provides a measure of affinity of the polymers of the invention for the bile acid, glycocholrate (GC) based on the amount of GC bound at a subsaturating concentration of 5mM (t = 0), and an estimate of the rate at which this bile acid dissociates into a large volume of buffer. The results are obtained as initial amounts of GC bound (t = 0) and amounts remaining bound after 2 minutes in buffer (t = 2 min); from these figures the % dissociation i.e. the proportion of bound GC dissociated from the polymer after 2 minutes can be obtained. The lower the % dissociation the more efficient the polymer can be expected to be in extracting bile acids in vivo.

Method

Test compound (150 mg) was equilibrated with 5mM sodium glycocholate (30 ml) in Krebs' buffer. The compound was separated by centrifugation and the total bound determined by subtraction of the amount in the supernatant from the total bile acid used. Dissociation was measured by resuspending the compound in Krebs, buffer, shaking and sampling the mixture through a filter at several time points up to 20 minutes. Radioactivity and hence bile acid dissociated was determined in the filtrate.

Results

The following % dissociation figures were obtained :

| Compound | % Dissociation |
|---|---|
| Cholestyramine | 45 |
| Example 1 | 20 |
| 4 | 5 |
| 6 | 5 |
| 7 | 5 |

**Claims**

1. A cross-linked polystyrene polymer of structure

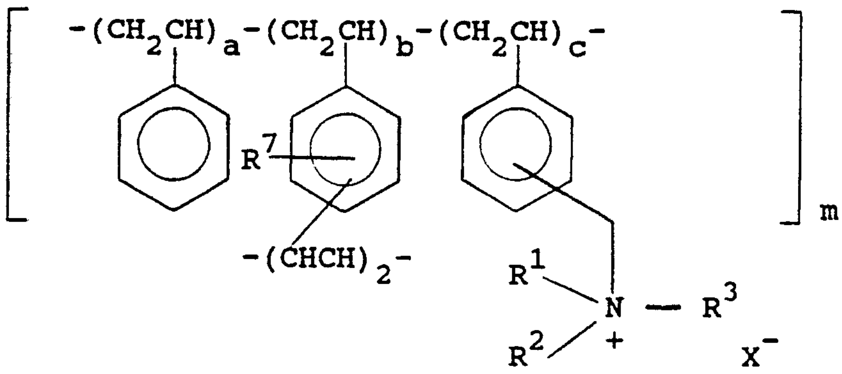

(I)

in which,
$R^1$ and $R^2$ are the same or different and are each $C_{1-4}$ alkyl;
$R^3$ is $(CH_2)_nR^4$ in which $R^4$ is $C_{3-6}$ cycloalkyl or a bicyclic carbocyclic ring of up to 10 carbon atoms and n is 1 to 12, or $R^3$ is $(CH_2)_nCH = R^5R^6$ in which n is 1 to 12 and $R^5$ and $R^6$ together form a $C_{3-6}$ cycloalkyl ring or a bicyclic carbocyclic ring of up to 10 carbon atoms;
$R^7$ is hydrogen or a group $CH_2\overset{+}{N}R^1R^2R^3X^-$;
$X^-$ is a counter ion;
a, b and c are numbers which indicate the relative molar percentages of the units present in said polymer, (b) being from about 1 to about 10 molar percent, and (c) being from about 30 to about 98 molar percent;
m is a number indicating the degree of polymerisation of said polymer.

2. A polystyrene polymers according to claim 1 in which $R^1$ and $R^2$ are each methyl.

3. A polystyrene polymers according to claim 2 in which (b) is from about 1 to about 4 molar percent of said polymer.

4. A process for the preparation of a polystyrene polymer according to any one of claims 1 to 3 which comprises

(a) reaction of a polymer of structure (II)

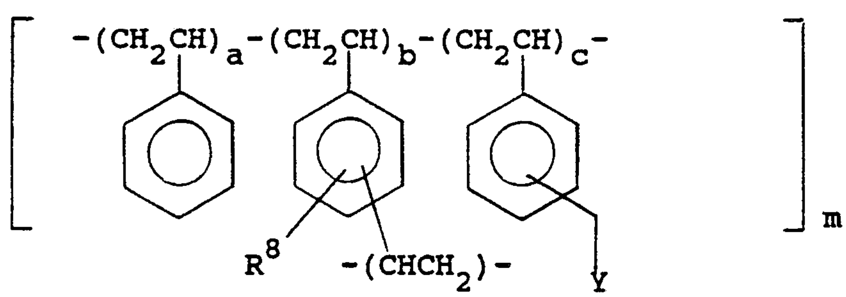

(II)

in which a, b, c and m are as described for structure (I), $R^8$ is $CH_2Y$ and Y is a group displaceable by an amine, with an amine of structure $R^1R^2NR^3$ (III) in which $R^1$ to $R^3$ are as described for structure (I); or

(b) reaction of a polymer of structure (IV)

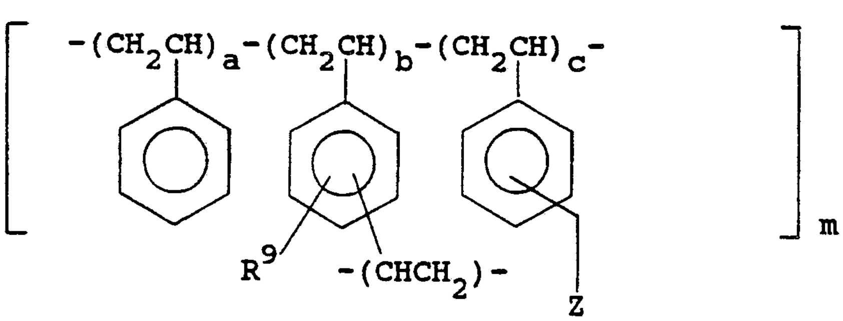

(IV)

in which a, b, c and m are as described for structure (I) $R^9$ is $CH_2Z$ and Z is $NR^1R^2$ or $NR^1R^3$ in which $R^1$ to $R^3$ are as described for structure (I), with a compound of structure $R^4Y$ (V) in which $R^4$ is $R^3$ when Z is $NR^1R^2$ or a group $R^2$ when Z is $NR^1R^3$, and Y is a group displaceable by an amine.

5. A pharmaceutical composition comprising a polystyrene polymer of structure (I) as claimed in claim 1 in association with a pharmaceutically acceptable carrier.

6. A polystyrene polymer according to claim 1 for use as a therapeutic agent.

7. A polystyrene polymer according to claim 1 for use in the lowering of serum cholesterol levels.